(19) **Europäisches Patentamt**
**European Patent Office**
**Office européen des brevets**

(11) Veröffentlichungsnummer: **0 061 114**
**B1**

# (12) EUROPÄISCHE PATENTSCHRIFT

(45) Veröffentlichungstag der Patentschrift: **12.12.84**

(51) Int. Cl.³: **C 07 D 213/64**, A 01 N 53/00, A 01 N 43/40

(21) Anmeldenummer: **82102071.6**

(22) Anmeldetag: **15.03.82**

(54) **Phenoxypyridylmethylester, Verfahren zu ihrer Herstellung und ihre Verwendung in Schädlingsbekämpfungsmitteln.**

(30) Priorität: **25.03.81 DE 3111644**

(43) Veröffentlichungstag der Anmeldung: **29.09.82 Patentblatt 82/39**

(45) Bekanntmachung des Hinweises auf die Patenterteilung: **12.12.84 Patentblatt 84/50**

(84) Benannte Vertragsstaaten: **BE CH DE FR GB IT LI NL**

(56) Entgegenhaltungen:
**EP - A - 0 037 851**
**GB - A - 2 079 282**
**US - A - 4 163 787**
**US - A - 4 183 942**
**US - A - 4 285 954**

**Die Akte enthält technische Angaben, die nach dem Eingang der Anmeldung eingereicht wurden und die nicht in dieser Patentschrift enthalten sind.**

(73) Patentinhaber: **BAYER AG, Konzernverwaltung RP Patentabteilung, D-5090 Leverkusen 1 Bayerwerk (DE)**

(72) Erfinder: **Fuchs, Rainer, Dr., Roeberstrasse 8, D-5600 Wuppertal 1 (DE)**
Erfinder: **Hammann, Ingeborg, Dr., Belfortstrasse 9, D-5000 Köln 1 (DE)**
Erfinder: **Homeyer, Bernhard, Dr., Obere Strasse 28, D-5090 Leverkusen 3 (DE)**
Erfinder: **Stendel, Wilhelm, Dr., In den Birken 55, D-5600 Wuppertal 1 (DE)**

EP 0 061 114 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

BUNDESDRUCKEREI BERLIN

**Beschreibung**

Die Erfindung betrifft neue Phenoxypyridylmethylester, ein Verfahren zu ihrer Herstellung und ihre Verwendung in Schädlingsbekämpfungsmitteln, insbesondere in Insektiziden und Akariziden.

Es ist bekannt, daß bestimmte Carbonsäureester, wie z. B. 3-(2-Chlor-2-phenyl-vinyl)-2,2-dimethyl-cyclopropancarbonsäure-(3-phenoxy-benzyl)-ester und 3-(2,2-Dichlorvinyl)-2,2-dimethyl-cyclopropancarbonsäure-(6-phenoxy-2-pyridyl-methyl)-ester, insektizide und akarizide Eigenschaften aufweisen (vergleiche US-PS 4 183 942 und 4 163 787). Die Wirkung dieser Verbindungen ist jedoch, insbesondere bei niedrigen Wirkstoffkonzentrationen und Aufwandmengen, nicht immer zufriedenstellend.

Es wurden nun neue Phenoxypyridylmethylester der Formel I

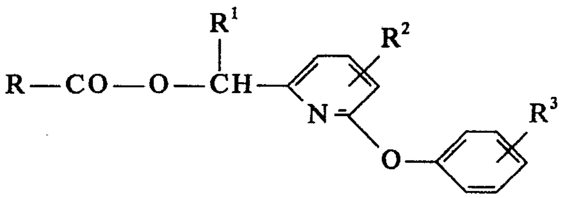

(I)

gefunden, in welcher

R für den Rest

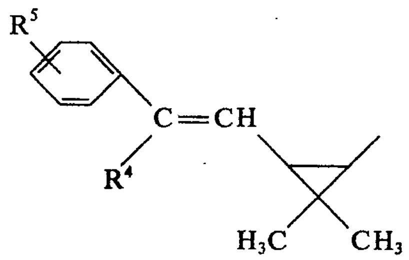

steht, worin

$R^4$ für Wasserstoff, Chlor oder gegebenenfalls Fluor oder Chlor substituiertes $C_{1-4}$-Alkyl steht und

$R^5$ für Wasserstoff oder Halogen steht

R weiter für den Rest

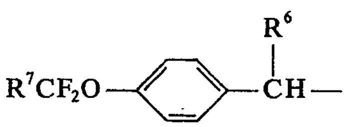

steht, worin

$R^6$ für Isopropyl steht und

$R^7$ für Wasserstoff, Fluor, Trifluormethyl oder Chlor(di)fluormethyl steht,

$R^1$ für Wasserstoff oder Cyano steht sowie

$R^2$ und $R^3$ gleich oder verschieden sind und für Wasserstoff oder Halogen stehen.

Die allgemeine Formel (I) schließt die verschiedenen möglichen Stereoisomeren und optischen Isomeren sowie deren Mischungen mit ein.

Man erhält die neuen Verbindungen der Formel (I), wenn man Carbonsäuren der Formel II

$$R—COOH \quad (II)$$

in welcher

R die oben angegebene Bedeutung hat,

oder reaktionsfähige Derivate derselben,

mit Phenoxypyridylalkoholen der Formel III

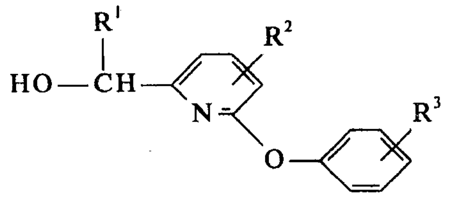

(III)

in welcher
$R^1$, $R^2$ und $R^3$ die oben angegebene Bedeutung haben,
oder mit reaktionsfähigen Derivaten derselben,
gegebenenfalls in Gegenwart von Säureakzeptoren, gegebenenfalls in Gegenwart von Katalysatoren, und gegebenenfalls in Gegenwart von Verdünnungsmitteln umsetzt.

Die neuen Phenoxypyridylmethylester der Formel (I) zeichnen sich durch hohe pestizide, insbesondere insektizide und akarizide Wirksamkeit aus.

Überraschenderweise zeigen die erfindungsgemäßen Verbindungen der Formel (I) eine erheblich höhere insektizide und akarizide Wirkung als aus dem Stand der Technik bekannte Verbindungen analoger Konstitution und gleicher Wirkungsrichtung.

Die Erfindung betrifft vorzugsweise Verbindungen der Formel (I), in welcher

R für den Rest

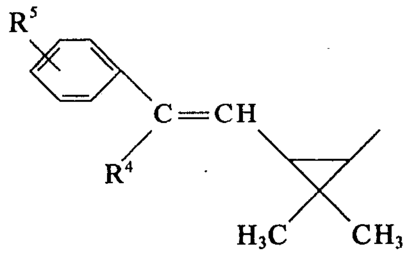

steht, worin

$R^4$ für Wasserstoff, Chlor oder gegebenenfalls durch Fluor oder Chlor substituiertes $C_1$-$C_4$-Alkyl steht und
$R^5$ für Wasserstoff oder Chlor steht,

R weiter für den Rest

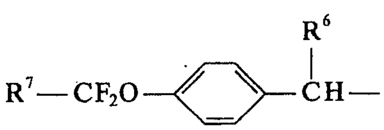

steht, worin

$R^6$ für Isoprophyl steht und
$R^7$ für Wasserstoff oder Fluor steht,

$R^1$ für Wasserstoff oder Cyano steht, sowie
$R^2$ und $R^3$ für Wasserstoff oder Fluor, stehen.

Die Erfindung betrifft insbesondere Verbindungen der Formel (I), in welcher

R für den Rest

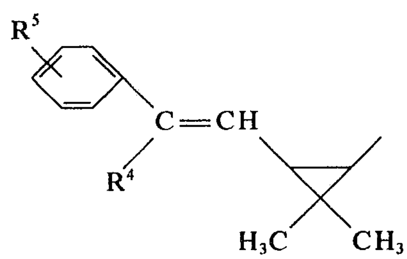

steht, worin

$R^4$ für Chlor steht,
$R^5$ für Wasserstoff oder Chlor (in para-Stellung) steht,

R weiter für den Rest

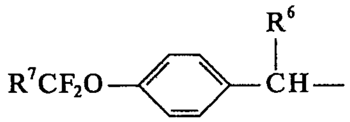

steht, worin

$R^6$ für Isopropyl steht und
$R^7$ für Fluor steht,

$R^1$ für Wasserstoff oder Cyano steht sowie
$R^2$ und $R^3$ für Wasserstoff stehen.

In einer bevorzugten Variante (a) des Herstellungsverfahrens für die Verbindungen der Formel (I) werden Carbonsäurechloride der Formel II a

$$R-CO-Cl \qquad (II\,a)$$

in welcher
R die oben angegebene Bedeutung hat,
mit Phenoxypyridylalkoholen der Formel III (oben) in Gegenwart von Säureakzeptoren und unter Verwendung von Verdünnungsmitteln umgesetzt.

In einer weiteren bevorzugten Variante (b) — zur Herstellung von Verbindungen der Formel (I), in welcher $R^1$ für Cyano steht — werden Carbonsäurechloride der Formel (II a) mit entsprechenden Phenoxypyridincarbaldehyden (Phenoxy-formyl-pyridinen) der Formel IV

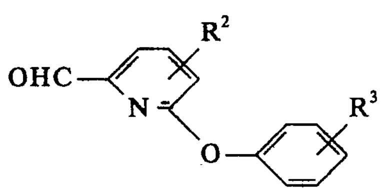

(IV)

in welcher
$R^2$ und $R^3$ die oben angegebene Bedeutung haben,
und wenigstens der äquimolaren Menge eines Alkalicyanids (Natrium- oder Kaliumcyanid) in Gegenwart von Wasser und eines mit Wasser nicht mischbaren organischen Lösungsmittels und gegebenenfalls in Gegenwart eines Katalysators umgesetzt.

Verwendet man als Ausgangsstoffe bei der Verfahrensvariante (a) beispielsweise 3-(2-Chlor-2-phenyl-vinyl)-2,2-dimethyl-cyclopropancarbonsäurechlorid und 6-Phenoxy-2-pyridyl-methanol, bei Variante (b) 3-Methyl-2-(4-difluormethoxy-phenyl)-butansäurechlorid, Natriumcyanid und 6-Phenoxy-pyridin-2-carbaldehyd, sol können die bei den beiden Verfahrensvarianten ablaufenden Reaktionen durch folgende Formelschemata skizziert werden:

(a) 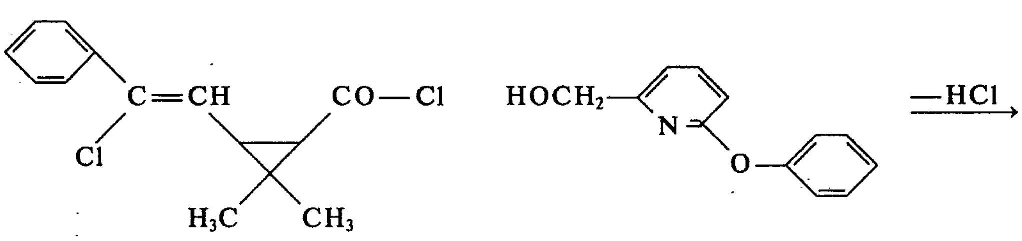

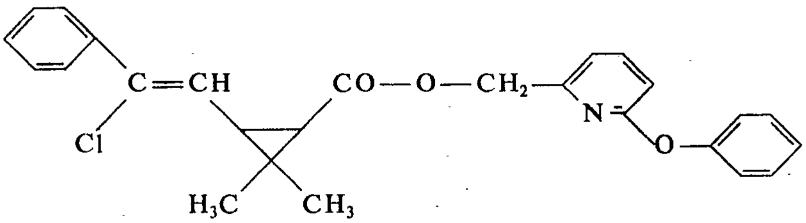

(b) 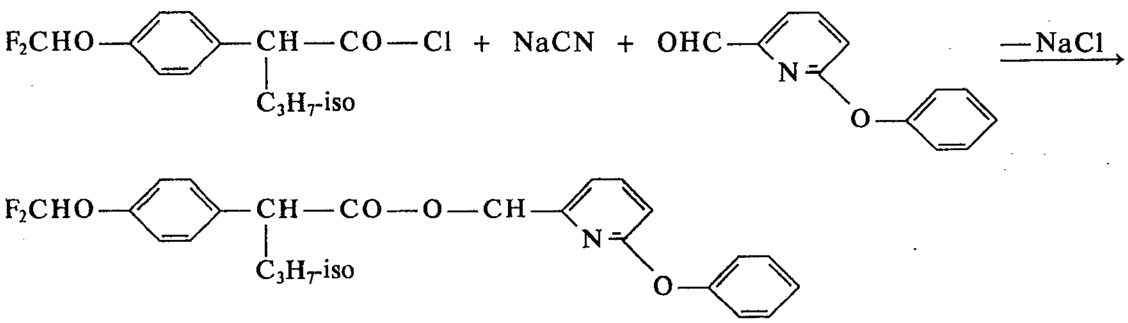


Die als Ausgangsstoffe zu verwendenden Carbonsäuren sind durch Formel (II), die entsprechenden Säurechloride durch Formel (II a) definiert.

R hat darin vorzugsweise bzw. insbesondere die gleiche Bedeutung, wie sie bei der Definition des entsprechenden Restes in Formel (I) vorzugsweise bzw. als insbesondere bevorzugt angegeben ist.

Als Beispiele für die Verbindungen der Formel (II a) seien genannt:

3-(2-Chlor-2-phenyl-vinyl)- und 3-(2-Chlor-2-(4-chlorphenyl)-vinyl)-2,2,-dimethyl-cyclopropancarbonsäurechlorid,
2-(4-Trifluormethoxyphenyl)- und 2-(4-Difluormethoxy-phenyl)-3-methyl-butansäurechlorid.

Verbindungen der Formeln (II) und (II a) sind bekannt (vergleiche US-PS 4 183 942 und 4 199 595).

Die weiter als Ausgangsstoffe zu verwendenden Phenoxypyridylalkohole sind durch Formel (III) definiert. Vorzugsweise bzw. insbesondere stehen darin $R^1$, $R^2$ und $R^3$ für diejenigen Reste, welche bereits bei der Definition von $R^1$, $R^2$ und $R^3$ in Formel (I) vorzugsweise bzw. als insbesondere bevorzugt genannt wurden.

Als Beispiele für die Ausgangsverbindungen der Formel (III) seien genannt:

6-Phenoxy-2-pyridylmethanol und 6-Phenoxy-2-pyridyl-$\alpha$-cyano-methanol.

Die Verbindungen der Formel (III) sind bereits bekannt (vergleiche US-PS 4 163 787).

Die als Ausgangsstoffe verwendbaren Phenoxypyridincarbaldehyde sind durch Formel (IV) definiert. $R^2$ und $R^3$ haben darin die gleiche bevorzugte bzw. insbesondere bevorzugte Bedeutung wie die entsprechenden Reste in Formel (I).

Als Beispiel sei 6-Phenoxy-pyridin-2-carbaldehyd genannt.

Die Verbindungen der Formel (IV) sind ebenfalls bekannt (vergleiche US-PS 4 163 787).

Das Verfahren zur Herstellung der neuen Verbindungen der Formel (I) wird in allen Varianten vorzugsweise unter Verwendung von Verdünnungsmitteln durchgeführt. Als Verdünnungsmittel kommen praktisch alle inerten organischen Lösungsmittel in Frage. Hierzu gehören insbesondere aliphatische und aromatische, gegebenenfalls halogenierte Kohlenwasserstoffe, wie Pentan, Hexan, Heptan, Cyclohexan, Petrolether, Benzin, Ligroin, Benzol, Toluol, Xylol, Methylenchlorid, Ethylenchlorid, Chloroform, Tetrachlorkohlenstoff, Chlorbenzol und o-Dichlorbenzol, Ether, wie Diethyl- und Dibutylether, Glycoldimethylether und Diglycoldimethylether, Tetrahydrofuran und Dioxan, Ketone, wie Aceton, Methylethyl-, Methyl-iso-propyl- und Methyl-iso-butyl-keton, Ester, wie Essigsäure-methylester und -ethylester, Nitrile, wie z. B. Acetonitril und Propionitril, Amide wie z. B. Dimethylformamid, Dimethylacetamid und N-Methyl-pyrrolidon, sowie Dimethylsulfoxid, Tetramethylensulfon und Hexamethylphosphorsäuretriamid.

Die Variante (a) des erfindungsgemäßen Verfahrens wird vorzugsweise in Gegenwart von Säureakzeptoren durchgeführt. Als Säureakzeptoren können die üblichen Säurebindemittel Verwendung finden. Besonders bewährt haben sich Alkalicarbonate und -alkoholate, wie Natrium- und Kaliumcarbonat, Natrium- und Kaliummethylat bzw. -ethylat, ferner aliphatische, aromatische oder heterocyclische Amine, beispielsweise Triethylamin, Trimethylamin, Dimethylanilin, Dimethylbenzylamin, Pyridin, Diazabicyclooctan, Diazabicyclononen und Diazabicycloundecen.

Die Variante (b) des erfindungsgemäßen Verfahrens wird in Gegenwart von Wasser und eines der oben genannten organischen Lösungsmittel, soweit mit Wasser nicht mischbar, durchgeführt. Hierfür

sind insbesondere die oben genannten Kohlenwasserstoffe geeignet.

Als Katalysatoren werden bei der Verfahrensvariante (b) vorzugsweise Verbindungen verwendet, welche zum Transfer von Anionen aus Wasser in organische Lösungsmittel geeignet sind. Beispiele hierfür sind Benzyl-triethylammoniumhydrogensulfat, Tetrabutylammonium-bromid und Methyl-trioctyl-ammonium-chlorid (Aliquat 336®).

In allen Verfahrensvarianten kann die Reaktionstemperatur innerhalb eines größeren Bereichs variiert werden. Im allgemeinen arbeitet man zwischen 0 und 100°C, vorzugsweise bei 10 bis 50°C.

Das erfindungsgemäße Verfahren wird im allgemeinen bei Normaldruck durchgeführt. Zur Durchführung des erfindungsgemäßen Verfahrens werden die Ausgangsstofe gewöhnlich in äquimolaren Mengen eingesetzt. Ein Überschuß der einen oder anderen Reaktionskomponente bringt keine wesentlichen Vorteile. Die Ausgangsstoffe werden in geeigneten Verdünnungsmitteln zusammen gegeben und, gegebenenfalls nach Zugabe eines Säureakzeptors und/oder eines Katalysators, bis zum Reaktionsende gerührt.

Die Aufarbeitung kann nach üblichen Methoden durchgeführt werden, beispielsweise indem man das Reaktionsgemisch gegebenenfalls mit Wasser und/oder einem mit Wasser nicht mischbaren organischen Lösungsmittel, wie z. B. Toluol, verdünnt, die organische Phase abtrennt, mit Wasser wäscht, trocknet, filtriert und vom Filtrat das Lösungsmittel unter vermindertem Druck und bei mäßig erhöhter Temperatur sorgfältig abdestilliert (»andestillieren«).

Die Wirkstoffe eignen sich bei guter Pflanzenverträglichkeit und günstiger Warmblütertoxizität zur Bekämpfung von tierischen Schädlingen, insbesondere Insekten, Spinnentieren, die in der Landwirtschaft, in Forsten, im Vorrats- und Materialschutz sowie auf dem Hygienesektor vorkommen. Sie sind gegen normal sensible und resistente Arten sowie gegen alle oder einzelne Entwicklungsstadien wirksam. Zu den oben erwähnten Schädlingen gehören:

Aus der Ordnung der Isopoda z. B. Oniscus asellus, Armadillidium vulgare, Porcellio scaber.

Aus der Ordnung der Diplopoda z. B. Blaniulus guttulatus.

Aus der Ordnung der Chilopoda z. B. Geophilus carpophagus, Scutigera spec.

Aus der Ordnung der Symphyla z. B. Scutigerella immaculata.

Aus der Ordnung der Thysanura z. B. Lepisma saccharina.

Aus der Ordnung der Collembola z. B. Onychiurus armatus.

Aus der Ordnung der Orthoptera z. B. Blatta orientalis, Periplaneta americana, Leucophaea maderae, Blattella germanica, Acheta domesticus, Gryllotalpa spp., Locusta migratoria migratorioides, Melanoplus differentialis, Schistocerca gregaria.

Aus der Ordnung der Dermaptera z. B. Forficula auricularia.

Aus der Ordnung der Isoptera z. B. Reticulitermes spp.

Aus der Ordnung der Anoplura z. B. Phylloxera vastatrix, Pemphigus spp., Pediculus humanus corporis, Haematopinus spp., Linognathus spp.

Aus der Ordnung der Mallophaga z. B. Trichodectes spp., Damalinea spp.

Aus der Ordnung der Thysanoptera z. B. Hercinothrips femoralis, Thrips tabaci.

Aus der Ordnung der Heteroptera z. B. Eurygaster spp., Dysdercus intermedius, Piesma quadrata, Cimex lectularius, Rhodnius prolixus, Triatoma spp.

Aus der Ordnung der Homoptera z. B. Aleurodes brassicae, Bemisia tabaci, Trialeurodes vaporariorum, Aphis gossypii, Brevicoryne brassicae, Cryptomyzus ribis, Doralis fabae, Doralis pomi, Eriosoma lanigerum, Hyalopterus arundinis, Macrosiphum avenae, Myzus spp., Phorodon humuli, Rhopalosiphum padi, Empoasca spp., Euscelis bilobatus, Nephotettix cincticeps, Lecanium corni, Saissetia oleae, Lacodelphax striatellus, Nilaparvata lugens, Aonidiella aurantii, Aspidiotus hederae, Pseudococcus spp. Psylla spp.

Aus der Ordnung der Lepidoptera z. B. Pectinophora gossypiella, Bupalus piniarius, Cheimatobia brumata, Lithocolletis blancardella, Hyponomeuta padella, Plutella maculipennis, Malacosoma neustria, Euproctis chrysorrhoea, Lymantria spp. Bucculatrix thurberiella, Phyllocnistis citrella, Agrotis spp., Euxoa spp., Feltia spp. Earias insulana, Heliothis spp., Laphygma exigua, Mamestra brassicae, Panolis flammea, Prodenia litura, Spodoptera spp., Trichoplusia ni, Carpocapsa pomonella, Pieris spp., Chilo spp., Pyrausta nubilalis, Ephestia kuehniella, Galleria mellonella, Cacoecia podana, Capua reticulana, Choristoneura fumiferana, Clysia ambiguella, Homona magnanima, Tortrix viridana.

Aus der Ordnung der Coleoptera z. B. Anobium punctatum, Rhizopertha dominica, Bruchidius obtectus, Acanthoscelides obtectus, Hylotrupes bajulus, Agelastica alni, Leptinotassa decemlineata, Phaedon cochleariae, Diabrotica spp., Psylliodes chrysocephala, Epilachna varivestis, Atomaria spp., Oryzaephilus surinamensis, Anthonomus spp., Sitophilus spp., Otiorrhynchus sulcatus, Cosmopolites sordidus, Ceuthorrhynchus assimilis, Hypera postica, Dermestes spp., Trogoderma spp., Anthrenus spp., Attagenus spp., Lyctus spp., Meligethes aeneus, Ptinus spp., Niptus holoeucus Gibbium psylloides, Tribolium spp., Tenebrio molitor, Agriotes spp., Conoderus spp., Melolontha melolontha, Amphimallon solstitialis, Costelytra zealandica.

Aus der Ordnung der Hymenoptera z. B. Diprion spp., Hoplocampa spp., Lasius spp., Monomorium pharaonis, Vespa spp.

Aus der Ordnung der Diptera z. B. Aedes spp., Anopheles spp., Culex spp., Drosophila melanogaster, Musca spp., Fannia spp., Calliphora erythrocephala, Lucilia spp., Chrysomyia spp., Cuterebra spp., Gastrophilus spp., Hyppobosca spp., Stomoxys spp., Oestrus spp., Hypoderma spp., Tabanus spp., Tannia spp., Bibio hortulanus, Oscinella frit, Phorbia spp., Pegomyia hyoscyami, Ceratitis capitata, Dacus oleae, Tipula paludosa.
Aus der Ordnung der Siphonaptera z. B. Xenopsylla cheopis, Ceratophyllus spp.
Aus der Ordnung der Arachnida z. B. Scorpio maurus, Latrodectus mactans.
Aus der Ordnung der Acarina z. B. Acarus siro, Argas spp., Ornithodoros spp., Dermanyssus gallinae, Eriophyes ribis, Phyllocoptruta oleivora, Boophilus spp., Rhipicephalus spp., Amblyomma spp., Hyalomma spp., Ixodes spp., Psoroptes spp., Chorioptes spp., Sarcoptes spp., Tarsonemus spp., Bryobia praetiosa, Panonychus spp., Tetranychus spp.

Die Wirkstoffe können in die üblichen Formulierungen übergeführt werden, wie Lösungen, Emulsionen, Suspensionen, Pulver, Schäume, Pasten, Granulate, Aerosole, Wirkstoff-imprägnierte Natur- und synthetische Stoffe, Feinstverkapselungen in polymeren Stoffen und in Hüllmassen für Saatgut, ferner in Formulierungen mit Brennsätzen, wie Räucherpatronen, -dosen, -spiralen u. ä., sowie ULV-Kalt- und Warmnebel-Formulierungen.

Diese Formulierungen werden in bekannter Weise hergestellt, z. B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z. B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chloräthylene oder Methylenchlorid, aliphatische Kohlenwasserstoffe, wie Cyclohexan oder Paraffine, z. B. Erdölfraktionen, Alkohole, wie Butanol oder Glycol sowie deren Äther und Ester, Ketone, wie Aceton, Methyläthylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z. B. Aerosol-Treibgas, wie Halogenkohlenwasserstoffe sowie Butan, Propan, Stickstoff und Kohlendioxid; als feste Trägerstoffe kommen in Frage: z. B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z. B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier- und/oder schaumerzeugende Mittel kommen in Frage: z. B. nichtiogene und anionische Emulgatoren, wie Polyoxyäthylen-Fettsäure-Ester, Polyoxyäthylen-Fettalkohol-Äther, z. B. Alkylarylpolyglykol-äther, Alkylsulfonate, Alkylsulfate, Arylsulfonate sowie Eiweißhydrolysate; als Dispergiermittel kommen in Frage: z. B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat.

Es können Farbstoffe wie anorganische Pigmente, z. B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90%.

Die erfindungsgemäßen Wirkstoffe können in ihren handelsüblichen Formulierungen sowie in den aus diesen Formulierungen bereiteten Anwendungsformen in Mischung mit anderen Wirkstoffen, wie Insektiziden, Lockstoffen, Sterilantien, Akariziden, Nematiziden, Fungiziden, wachstumsregulierenden Stoffen oder Herbiziden vorliegen. Zu den Insektiziden zählen beispielsweise Phosphorsäureester, Carbamate, Carbonsäureester, chlorierte Kohlenwasserstoffe, Phenylharnstoffe, durch Mikroorganismen hergestellte Stoffe u. a.

Die erfindungsgemäßen Wirkstoffe können ferner in ihren handelsüblichen Formulierungen sowie in den aus diesen Formulierungen bereiteten Anwendungsformen in Mischung mit Synergisten vorliegen. Synergisten sind Verbindungen, durch die die Wirkung der Wirkstoffe gesteigert wird, ohne daß der zugesetzte Synergist selbst aktiv wirksam sein muß.

Der Wirkstoffgehalt der aus den handelsüblichen Formulierungen bereiteten Anwendungsformen kann in weiten Bereichen variieren. Die Wirkstoffkonzentration der Anwendungsformen kann von 0,0000001 bis zu 100 Gew.-% Wirkstoff, vorzugsweise zwischen 0,0001 und 1 Gew.-% liegen.

Die Anwendung geschieht in einer den Anwendungsformen angepaßten üblichen Weise.

Bei der Anwendung gegen Hygiene- und Vorratsschädlinge zeichnen sich die Wirkstoffe durch eine hervorragende Residualwirkung auf Holz und Ton sowie durch eine gute Alkalistabilität auf gekälkten Unterlagen aus.

Die erfindungsgemäßen Wirkstoffe eignen sich auch zur Bekämpfung von Ekto- und Endoparasiten auf dem veterinärmedizinischen Gebiet.

Die Anwendung der erfindungsgemäßen Wirkstoffe geschieht im Veterinärsektor in bekannter Weise, wie durch orale Anwendung in Form von beispielsweise Tabletten, Kapseln, Tränken, Granulaten, durch dermale Anwendung in Form beispielsweise des Tauchens (Dippen), Sprühens (Sprayen), Aufgießens (pour-on and spot-on) und des Einpuderns sowie durch parenterale Anwendung in Form beispielsweise der Injektion.

## Herstellungsbeispiele

### Beispiel 1

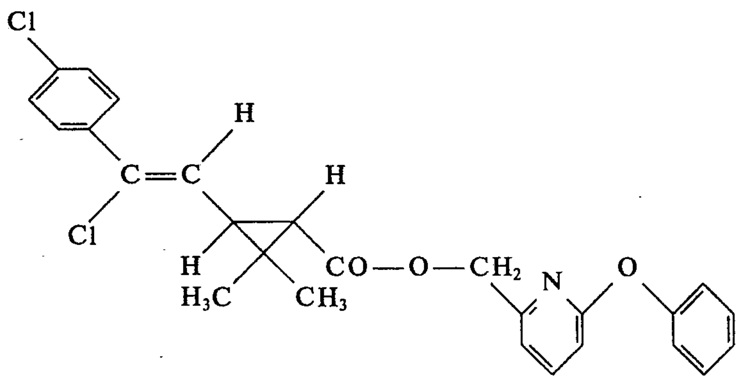

4,02 g (0,02 Mol) (6-Phenoxy-2-pyridinyl)-methanol und 6,07 g (0,02 Mol) (±)-trans-Z-3-(2-Chlor-2-(4-chlorphenyl)-vinyl)-2,2-dimethyl-1-cyclopropancarbonsäurechlorid werden in 100 ml wasserfreiem Toluol gelöst und bei 20—25°C 2 g Pyridin, gelöst in 10 ml wasserfreiem Toluol, unter Rühren zugetropft. Anschließend wird weitere 3 Stunden bei 25°C gerührt. Das Reaktionsgemisch wird in 150 ml Wasser gegossen, die organische Phase abgetrennt und nochmals mit 100 ml Wasser gewaschen. Anschließend wird die Toluolphase über Natriumsulfat getrocknet und das Lösungsmittel im Wasserstrahlvakuum abdestilliert. Letzte Lösungsmittelreste werden durch kurzes Andestillieren bei 60°C/1 Torr Badtemperatur entfernt. Man erhält 8,6 g (92% der Theorie) (±)-trans-Z-3-(2-Chlor-2-(4-chlorphenyl)-vinyl)-2,2-dimethyl-1-cyclopropancarbonsäure-(6-phenoxy-2-pyridinyl)-methylester als gelbes zähes Öl.

Die Struktur wird durch das $^1$H-NMR-Spektrum bewiesen.

$^1$H-NMR in $CDCl_3$/TMS $\tau$ (ppm):

| | |
|---|---|
| aromatische-H: | 2,2—3,4 (m/12 H) |
| vinyl-H: | 4,15 (S/1 H) |
| benzyl-H: | 4,88 (S/2 H) |
| cyclopropan-H: | 7,28—7,58 (m/1 H) |
| cyclopropan-H: | 8,17 (d/1 H) |
| dimethyl-H: | 8,6 (S/3 H) |
| dimethyl-H: | 8,74 (S/3 H) |

### Beispiel 2

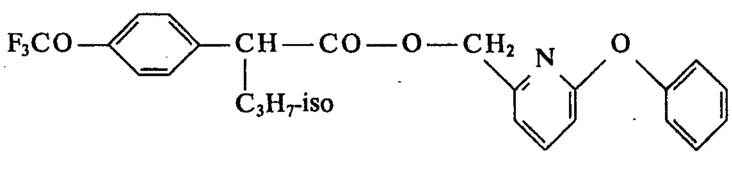

4,02 g (0,02 Mol) (6-Phenoxy-2-pyridinyl)-methanol und 5,6 g (0,02 Mol) $\alpha$-Isopropyl-4-trifluormethoxy-phenylessigsäurechlorid werden in 100 ml wasserfreiem Toluol gelöst und bei 20—25°C 2 g Pyridin, gelöst in 20 ml wasserfreiem Toluol, unter Rühren zugetropft. Anschließend wird weitere 3 Stunden bei 25°C gerührt. Das Reaktionsgemisch wird in 150 ml Wasser gegossen, die organische Phase abgetrennt und nochmals mit 100 ml Wasser gewaschen. Anschließend wird die Toluolphase über Natriumsulfat getrocknet und das Lösungsmittel im Wasserstrahlvakuum abdestilliert. Letzte Lösungsmittelreste werden durch kurzes Andestillieren bei 60°C/1 Torr Badtemperatur entfernt. Man

erhält 7,6 g (85,4% der Theorie) α-Isopropyl-4-trifluormethoxy-phenylessigsäure-(6-phenoxy-2-pyridinyl)-methylester als gelbes zähes Öl. Die Struktur wird durch das $^1$H-NMR bewiesen.

$^1$H-NMR- in $CDCl_1$/TMS τ (ppm):

aromatische-H: 2,32–3,38 (m/8 H)
benzyl-H: 4,9 (S/2 H)

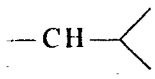 6,71 (d/1 H)

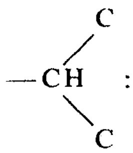 : 7,43–7,81 (m/1 H)

dimethyl-H: 8,88–9,31 (m/6 H).

Beispiel 3

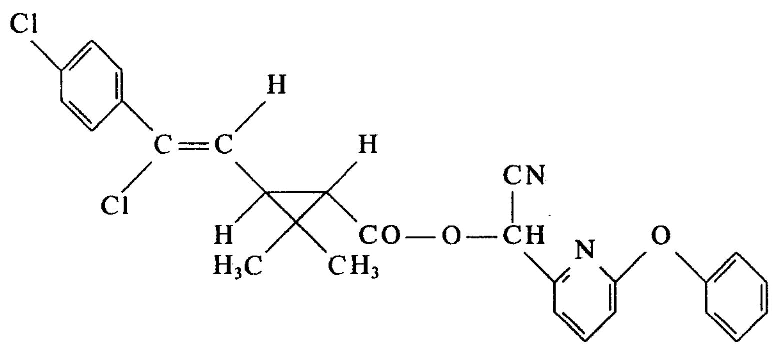

Zu einer Mischung von 40 ml n-Hexan, 1,5 g Natriumcyanid, 2 ml Wasser, 3,98 g (0,02 Mol) 2-Formyl-6-phenoxy-pyridin und 0,5 g Tetrabutylammoniumbromid werden unter Rühren bei 20–25°C 6,7 g (0,02 Mol) (±)-trans-Z-3-(2-Chlor-2-(4-chlor-phenyl)-vinyl)-2,2-dimethyl-1-cyclopropancarbonsäurechlorid gelöst in 10 ml n-Hexan zugetropft und dann 4 Stunden bei 20–25°C gerührt. Anschließend wird die Reaktionsmischung mit 100 ml Toluol versetzt und 2mal mit je 60 ml Wasser ausgeschüttelt. Die organische Phase wird abgetrennt, über Magnesiumsulfat getrocknet und das Lösungsmittel im Wasserstrahlvakuum abdestilliert. Letzte Lösungsmittelreste werden durch kurzes Andestillieren bei 60°C/1 Torr Badtemperatur entfernt. Man erhält 6,4 g (77% der Theorie) (±)-trans-Z-3-(2-Chlor-2-(4-chlor-phenyl)-vinyl)-2,2-dimethylcyclopropancarbonsäure-cyano-(6-phenoxy-2-pyridinyl)-methylester als zähes gelbes Öl. Die Struktur wird durch das $^1$H-NMR-Spektrum bewiesen.

$^1$H-NMR in $CDCl_3$/TMS τ (ppm):

Vinyl-H: 4,15 (s/1 H)

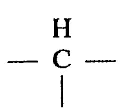 CN:3,6–3,65 (m/1 H)

Beispiel 4

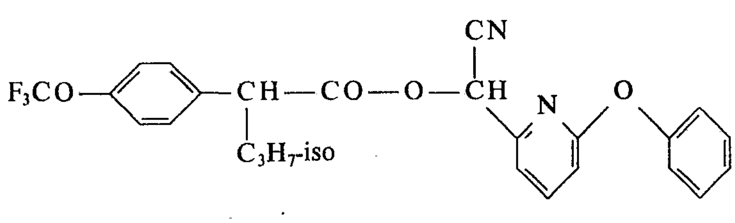

Zu einer Mischung von 50 ml n-Hexan, 1,5 g Natriumcyanid, 2 ml Wasser, 3,98 g (0,02 Mol) 2-Formyl-6-phenoxy-pyridin und 0,5 g Tetrabutylammoniumbromid werden unter Rühren bei 20–25°C 5,6 g (0,02 Mol) $\alpha$-Isopropyl-4-trifluormethoxy-phenylessigsäurechlorid, gelöst in 10 ml n-Hexan zugetropft und dann 4 Stunden bei 20–25°C gerührt. Anschließend wird die Reaktionsmischung mit 100 ml Toluol versetzt und 2mal mit je 60 ml Wasser ausgeschüttelt. Die organische Phase wird abgetrennt, über Magnesiumsulfat getrocknet und das Lösungsmittel im Wasserstrahlvakuum abdestilliert. Letzte Lösungsmittelreste werden durch kurzes Andestillieren bei 60°C/1 Torr Badtemperatur entfernt. Man erhält 7,8 g $\alpha$-Isopropyl-4-trifluormethoxy-phenyl-essigsäure-cyano-(6-phenoxy-2-pyridinyl)-methylester als gelbes zähes Öl. Die Struktur wird durch das $^1$H-NMR-Spektrum bewiesen.

$^1$H-NMR in $CDCl_3$/ $\tau$ (ppm):

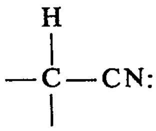: 3,6–3,65 (M/1 H)

Analog den Beispielen 1–4 können noch folgende Beispiele dargestellt werden.

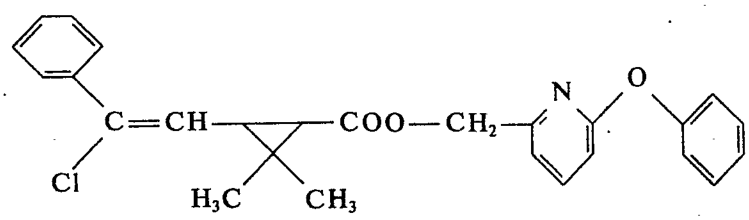

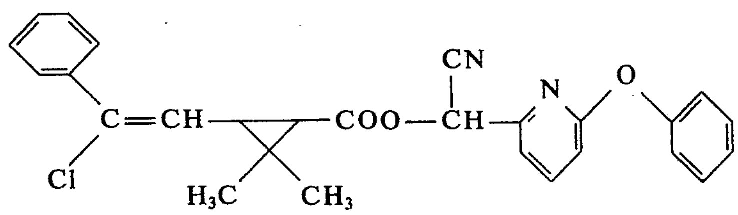

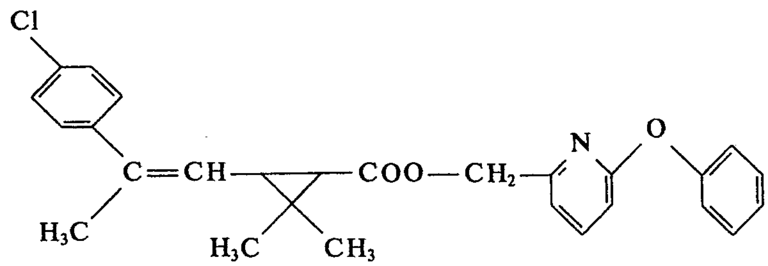

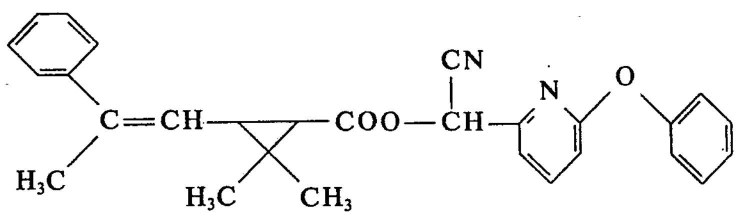

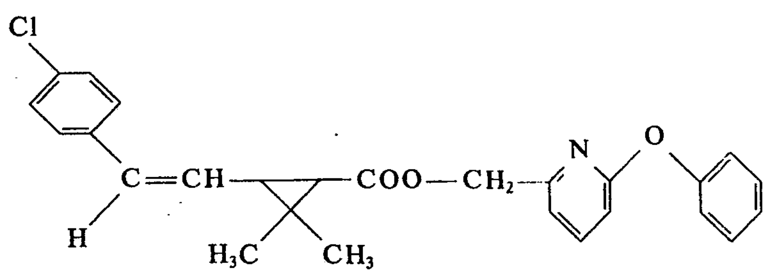

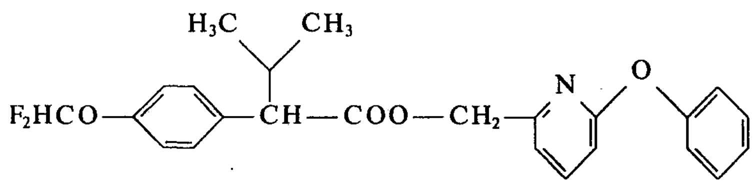

## Beispiel A

### Phaedon-Larven-Test

Lösungsmittel: 3 Gewichtsteile Aceton
Emulgator: 1 Gewichtsteil Alkylarylpolyglykoläther

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Kohlblätter (Brassica oleracea) werden durch Tauchen in die Wirkstoffzubereitung der gewünschten Konzentration behandelt und mit Meerrettichblattkäfer-Larven (Phaedon cochleariae) besetzt, solange die Blätter noch feucht sind.

Nach der gewünschten Zeit wird die Abtötung in % bestimmt. Dabei bedeutet 100%, daß alle Käfer-Larven abgetötet wurden; 0% bedeutet, daß keine Käfer-Larven abgetötet wurden.

Bei diesem Test zeigen z. B. die folgenden Verbindungen der Herstellungsbeispiele überlegene Wirksamkeit gegenüber dem Stand der Technik: 1 und 2.

## Beispiel B

### Tetranychus-Test (resistent)

Lösungsmittel: 3 Gewichtsteile Aceton
Emulgator: 1 Gewichtsteil Alkylarylpolyglykoläther

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Bohnenpflanzen (Phaseolus vulgaris), die stark von allen Entwicklungsstadien der gemeinen Spinnmilbe oder Bohnenspinnmilbe (Tetranychus urticae) befallen sind, werden durch Tauchen in die Wirkstoffzubereitung der gewünschten Konzentration behandelt.

Nach der gewünschten Zeit wird die Abtötung in % bestimmt. Dabei bedeutet 100%, daß alle Spinnmilben abgetötet wurden; 0% bedeutet, daß keine Spinnmilben abgetötet wurden.

Bei diesem Test zeigen z. B. die folgenden Verbindungen der Herstellungsbeispiele überlegene Wirksamkeit gegenüber dem Stand der Technik: 1 und 2.

## Beispiel C

### Grenzkonzentrations-Test/Bodeninsekten

Testinsekt: Agrotis segetum-Larven (im Boden)

Lösungsmittel: 3 Gewichtsteile Aceton
Emulgator: 1 Gewichtsteil Alkylarylpolyglykoläther

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Die Wirkstoffzubereitung wird innig mit dem Boden vermischt. Dabei spielt die Konzentration des Wirkstoffs in der Zubereitung praktisch keine Rolle, entscheidend ist allein die Wirkstoffgewichtsmenge pro Volumeneinheit Boden, welche in ppm (= mg/l) angegeben wird. Man füllt den Boden in Töpfe und läßt diese bei Raumtemperatur stehen.

Nach 24 Stunden werden die Testtiere in den behandelten Boden gegeben, und nach weiteren 2 bis 7 Tagen wird der Wirkungsgrad des Wirkstoffs durch Auszählen der toten und lebenden Testinsekten in % bestimmt. Der Wirkungsgrad ist 100%, wenn alle Testinsekten abgetötet worden sind, er ist 0%,

wenn noch genau so viele Testinsekten leben wie bei der unbehandelten Kontrolle.

Bei diesem Test zeigen z. B. die folgenden Verbindungen der Herstellungsbeispiele überlegene Wirkung gegenüber dem Stand der Technik: 1 und 2.

## Beispiel D

### Grenzkonzentrations-Test/Bodeninsekten

Testinsekt: Phorbia antiqua-Maden (im Boden)

Lösungsmittel: 3 Gewichtsteile Aceton
Emulgator: 1 Gewichtsteil Alkylarylpolyglykoläther

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Die Wirkstoffzubereitung wird innig mit dem Boden vermischt. Dabei spielt die Konzentration des Wirkstoffs in der Zubereitung praktisch keine Rolle, entscheidend ist allein die Wirkstoffgewichtsmenge pro Volumeneinheit Boden, welche in ppm (= mg/l) angegeben wird. Man füllt den Boden in Töpfe und läßt diese bei Raumtemperatur stehen.

Nach 24 Stunden werden die Testtiere in den behandelten Boden gegeben, und nach weiteren 2 bis 7 Tagen wird der Wirkungsgrad des Wirkstoffs durch Auszählen der toten und lebenden Testinsekten in % bestimmt. Der Wirkungsgrad ist 100%, wenn alle Testinsekten abgetötet worden sind, er ist 0%, wenn noch genau so viele Testinsekten leben wie bei der unbehandelten Kontrolle.

Bei diesem Test zeigen z. B. die folgenden Verbindungen der Herstellungsbeispiele überlegene Wirkung gegenüber dem Stand der Technik: 1 und 2.

## Beispiel E

### Test mit Boophilus microplus resistent

Lösungsmittel: 35 Gewichtsteile Äthylenglykolmonomethyläther
35 Gewichtsteile Nonylphenolpolyglykoläther

Zur Herstellung einer zweckmäßigen Wirkstoffzubereitung vermischt man drei Gewichtsteile Wirkstoff mit sieben Gewichtsteilen des oben angegebenen Lösungsmittel-Gemisches und verdünnt das so erhaltene Konzentrat mit Wasser auf die gewünschte Konzentration.

10 adulte Boophilus microplus res. werden in die zu testende Wirkstoffzubereitung 1 Minute getaucht. Nach Überführung in Plastikbecher und Aufbewahrung in einem klimatisierten Raum wird der Abtötungsgrad bestimmt.

Bei diesem Test zeigen z. B. die folgenden Verbindungen der Herstellungsbeispiele überlegene Wirkung gegenüber dem Stand der Technik: 1 und 2.

## Beispiel F

### Test mit Lucilia cuprina res.-Larven

Emulgator: 35 Gewichtsteile Äthylenglykolmonomethyläther
35 Gewichtsteile Nonylphenolpolyglykoläther

Zur Herstellung einer zweckmäßigen Wirkstoffzubereitung vermischt man drei Gewichtsteile Wirkstoff mit sieben Gewichtsteilen des oben angegebenen Lösungsmittel-Gemisches und verdünnt das so erhaltene Konzentrat mit Wasser auf die jeweils gewünschte Konzentration.

Etwa 20 Lucilia cuprina res.-Larven werden in ein Teströhrchen gebracht, welches ca. 1 $cm^2$ Pferdefleisch und 0,5 ml der Wirkstoffzubereitung enthält. Nach 24 Stunden wird der Abtötungsgrad bestimmt.

Bei diesem Test zeigen z. B. die folgenden Verbindungen der Herstellungsbeispiele überlegene Wirkung gegenüber dem Stand der Technik: 1 und 2.

**Patentansprüche**

1. Phenoxypyridylmethylester der Formel (I)

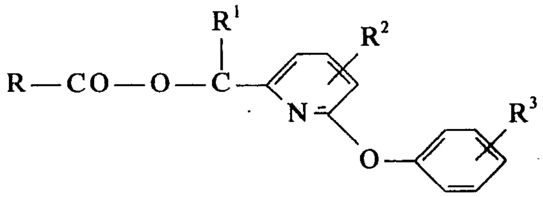

(I)

in welcher

R für den Rest

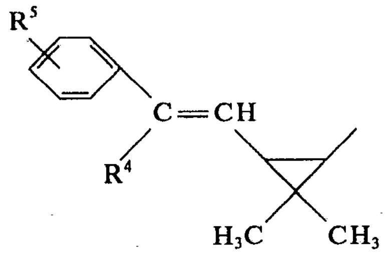

steht, worin

$R^4$ für Wasserstoff, Chlor oder gegebenenfalls fluor- oder chlorsubstituiertes $C_{1-4}$-Alkyl steht und
$R^5$ für Wasserstoff oder Halogen steht

R weiter für den Rest

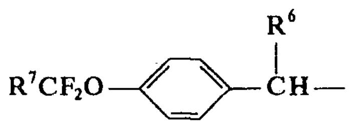

steht, worin

$R^6$ für Isopropyl steht und
$R^7$ für Wasserstoff, Fluor, Trifluormethyl oder Chlor-(di)-fluormethyl steht,

$R^1$ für Wasserstoff oder Cyano steht, sowie
$R^2$ und $R^3$ gleich oder verschieden sind und für Wasserstoff oder Halogen stehen.

2. Verfahren zur Herstellung der Phenoxypyridylmethylester der Formel (I)

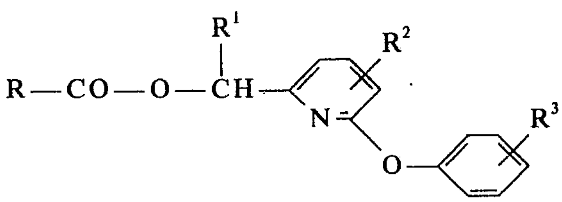

(I)

in welcher

R für den Rest

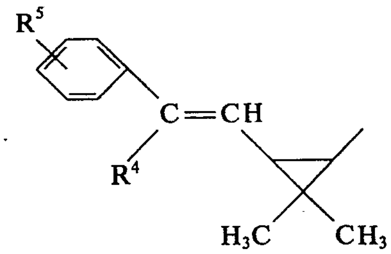

steht, worin

$R^4$ für Wasserstoff, Chlor oder gegebenenfalls fluor- oder chlor-substituiertes $C_{1-4}$-Alkyl steht und

$R^5$ für Wasserstoff oder Halogen steht

R weiter für den Rest

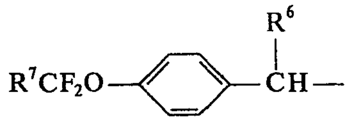

steht, worin

$R^6$ für Isopropyl steht und

$R^7$ für Wasserstoff, Fluor, Trifluormethyl oder Chlor-(di)fluormethyl steht,

$R^1$ für Wasserstoff oder Cyano steht sowie

$R^2$ und $R^3$ gleich oder verschieden sind und für Wasserstoff oder Halogen stehen,

dadurch gekennzeichnet, daß man Carbonsäuren der Formel (II)

$$R-COOH \qquad \text{(II)}$$

in welcher
R die oben angegebene Bedeutung hat,
oder reaktionsfähige Derivate derselben,
mit Phenoxypyridylalkoholen der Formel (III)

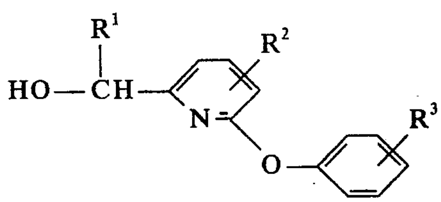

(III)

in welcher
$R^1$, $R^2$ und $R^3$ die oben angegebene Bedeutung haben,
oder mit reaktionsfähigen Derivaten derselben,
gegebenenfalls in Gegenwart von Säureakzeptoren, gegebenenfals in Gegenwart von Katalysatoren und gegebenenfalls in Gegenwart von Verdünnungsmitteln umsetzt.

3. Phenoxypyridylmethyester der Formel (I) gemäß Anspruch 1,
in welcher

R für den Rest

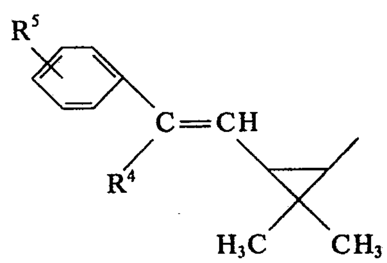

steht, worin

$R^4$ für Wasserstoff, Chlor oder gegebenenfalls durch Fluor oder Chlor substituiertes $C_1-C_4$-Alkyl steht und
$R^5$ für Wasserstoff oder Chlor steht,

R weiter für den Rest

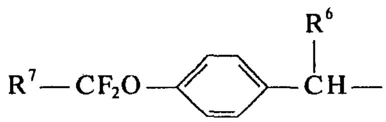

steht, worin
$R^6$ für Isopropyl steht und
$R^7$ für Wasserstoff oder Fluor steht,
$R^1$ für Wasserstoff oder Cyano steht
sowie
$R^2$ und $R^3$ für Wasserstoff oder Fluor stehen.

4. Phenoxypyridylmethylester der Formel (I) gemäß Anspruch 1, in welcher

R für den Rest

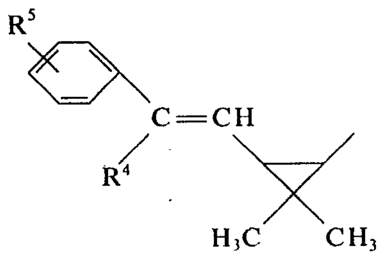

steht, worin

$R^4$ für Chlor steht,
$R^5$ für Wasserstoff oder Chlor (in para-Stellung) steht,

R weiter für den Rest

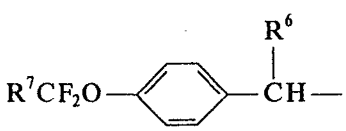

steht, worin

$R^6$ für Isopropyl steht und
$R^7$ für Fluor steht,

$R^1$ für Wasserstoff oder Cyano steht sowie
$R^2$ und $R^3$ für Wasserstoff stehen.

5. Schädlingsbekämpfungsmittel, gekennzeichnet durch einen Gehalt an mindestens einem Phenoxypyridylmethylester der Formel (I).
6. Verwendung von Phenoxypyridylmethylester der Formel (I) zur Bekämpfung von Schädlingen.
7. Verfahren zur Bekämpfung von Schädlingen, dadurch gekennzeichnet, daß man Phenoxypyridylmethylester der Formel (I) auf Schädlinge und/oder ihren Lebensraum einwirken läßt.
8. Verfahren zur Herstellung von Schädlingsbekämpfungsmitteln, dadurch gekennzeichnet, daß man Phenoxypyridylmethylester der Formel (I) mit Streckmitteln und/oder oberflächenaktiven Mitteln vermischt.

**Claims**

1. Phenoxypyridylmethylesters of the formula (I)

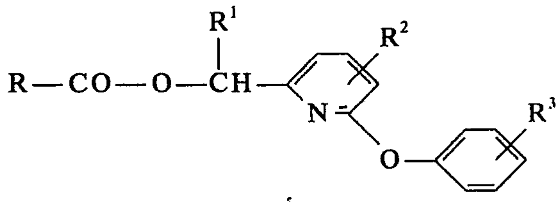

(I)

in which

R represents the radical

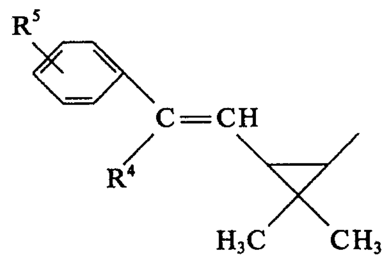

wherein

$R^4$ represents hydrogen, chlorine or optionally fluorine- or chlorine-substituted $C_{1-4}$-alkyl and
$R^5$ represents hydrogen or halogen,

R further represents the radical

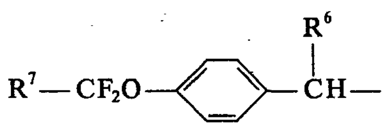

wherein

$R^6$ represents iospropyl and
$R^7$ represents hydrogen, fluorine, trifluoromethyl or chloro-(di)fluoromethyl,

$R^1$ represents hydrogen or cyano, and
$R^2$ and $R^3$ are identical or different and represent hydrogen or halogen.

2. Process for the preparation of the phenoxypyridylmethyl esters of the formula (I)

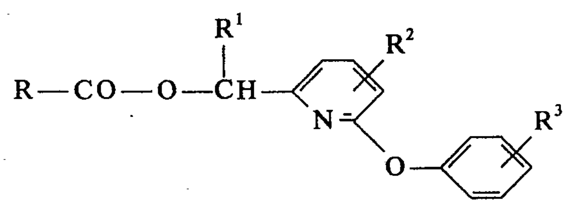

(I)

in which

R represents the radical

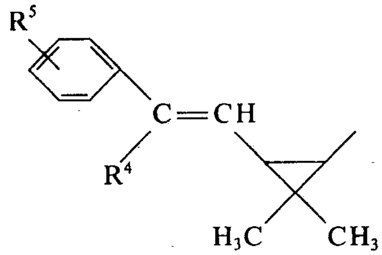

wherein

$R^4$ represents hydrogen, chlorine or optionally fluorine- or chlorine-substituted $C_{1-4}$-alkyl and
$R^5$ represents hydrogen or halogen,

R further represents the radical

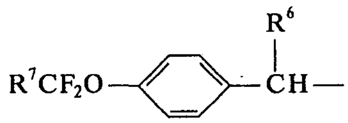

wherein
$R^6$ represents isopropyl and
$R^7$ represents hydrogen, fluorine, trifluoromethyl or chloro-(di)fluoromethyl,

$R^1$ represents hydrogen or cyano and
$R^2$ and $R^3$ are identical or different and represent hydrogen or halogen,

characterised in that carboxylic acids of the formula (II)

$$R—COOH \qquad (II)$$

in which
R has the meaning indicated above,
or reactive derivatives there of,
are reacted with phenoxypyridyl alcohols of the formula (III)

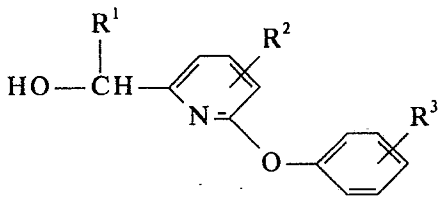

(III)

in which
$R^1$, $R^2$ and $R^3$ have the meaning indicated above, or with reactive derivatives thereof, if appropriate in the presence of acid acceptors, if appropriate in the presence of catalysts and if appropriate in the presence of diluents.

3. Phenoxypyridylmethyl esters of the formula (I) according to Claim 1, in which

R represents the radical

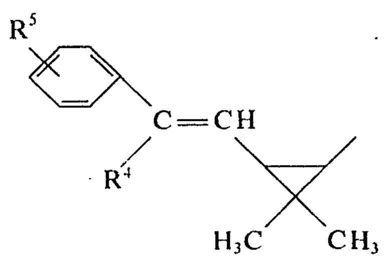

wherein,

$R^4$ represents hydrogen, chlorine or $C_1$—$C_4$-alkyl which is optionally substituted by fluorine or chlorine and
$R^5$ represents hydrogen or chlorine,

R further represents the radical

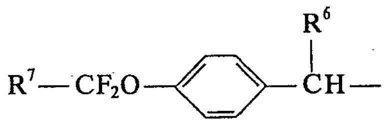

wherein

$R^6$ represents isopropyl and
$R^7$ represents hydrogen or fluorine,

$R^1$ represents hydrogen or cyano and
$R^2$ and $R^3$ represent hydrogen or fluorine.

4. Phenoxypyridylmethyl esters of the formula (I) according to Claim 1, in which

R represents the radical

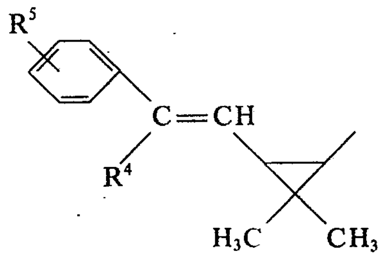

wherein

$R^4$ represents chlorine,
$R^5$ represents hydrogen or chlorine (in the para position),

R further represents the radical

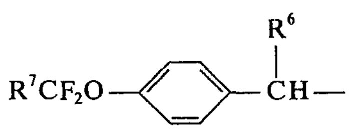

wherein

$R^6$ represents isopropyl and
$R^7$ represents fluorine,

$R^1$ represents hydrogen or cyano and
$R^2$ and $R^3$ represent hydrogen,

5. Agents for combating pests, characterised in that they contain at least one phenoxypyridylmethylester of the formula (I).

6. Use of phenoxypyridylmethyl esters of the formula (I) for combating pests.

7. Method of combating pests, characterised in that phenoxypyridylmethyl esters of the formula (I) are allowed to act on pests and/or their habitat.

8. Process for the preparation of agents for combating pests, characterised in that phenoxypyridylmethyl esters of the formula (I) are mixed with extenders and/or surface-active agents.

**Revendications**

1. Esters phénoxypyridylméthyliques de formule I

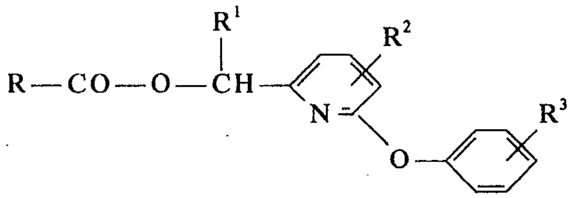 (I)

dans laquelle

R représente le reste

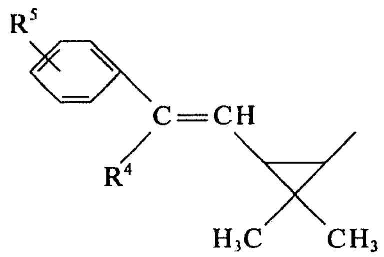

dans lequel

$R^4$ représente l'hydrogène, le chlore ou un groupe alkyle en $C_1$—$C_4$ éventuellement substitué par le fluor ou le chlore, et
$R^5$ représente l'hydrogène ou un halogène,

R représente en outre le reste

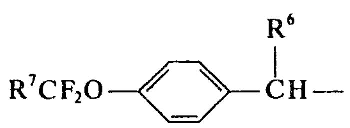

dans lequel

$R^6$ représente le groupe isopropyle, et
$R^7$ représente l'hydrogène, le fluor, un groupe trifluorométhyle ou chloro-(di)-fluorométhyle,

$R^1$ représente l'hydrogène ou le groupe cyano, et
$R^2$ et $R^3$ ayant des significations identiques ou différentes, représentent l'hydrogène ou un halogène.

2. Procédé de préparation des esters phénoxypyridylméthyliques de formule I

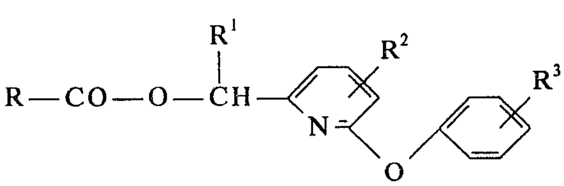 (I)

dans laquelle

R représente le reste

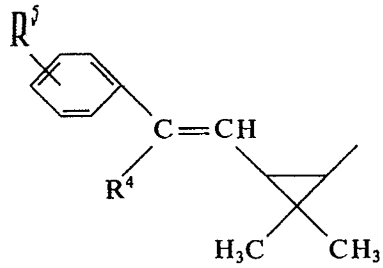

dans lequel

$R^4$ représente l'hydrogène, le chlore ou un groupe alkyle en $C_1$—$C_4$ éventuellement substitué par le fluor ou le chlore, et
$R^5$ représente l'hydrogène ou un halogène,

R représente en outre le reste

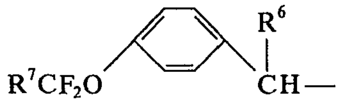

dans lequel

$R^6$ représente un groupe isopropyle, et
$R^7$ représente l'hydrogène, le fluor, un groupe trifluorméthyle ou chloro-(di)-fluorométhyle,

$R^1$ représente l'hydrogène ou un groupe cyano, et
$R^2$ et $R^3$ ayant des significations identiques ou différentes, représentent l'hydrogène ou un halogène,

caractérisé en ce que l'on fait réagir des acides carboxyliques de formule II

$$R—COOH \quad (II)$$

dans laquelle
R a la signification indiquée ci-dessus,
ou des dérivés réactifs de tels acides,
avec des alcools phénoxypyridyliques de formule III

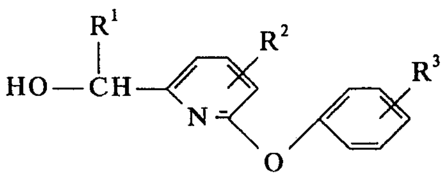

(III)

dans laquelle
$R^1$, $R^2$ et $R^3$ ont les significations indiquées ci-dessus,
ou avec des dérivés réactifs de tels alcools,
éventuellement en présence d'accepteurs d'acides, éventuellement en présence de catalyseurs et éventuellement en présence de diluants.

3. Esters phénoxypyridylméthyliques de formule I selon la revendication 1,
dans lesquels

R représente le reste

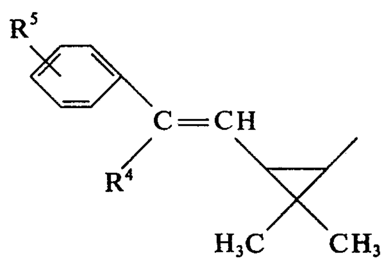

dans lequel

$R^4$ représente l'hydrogène, le chlore ou un groupe alkyle en $C_1-C_4$ éventuellement substitué par le fluor ou le chlore, et

$R^5$ représente l'hydrogène ou le chlore,

R représente en outre le reste

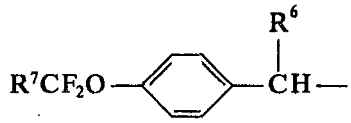

dans lequel

$R^6$ représente un groupe isopropyle, et

$R^7$ représente l'hydrogène ou le fluor,

$R^1$ représente l'hydrogène ou un groupe cyano, et

$R^2$ et $R^3$ représentent l'hydrogène ou le fluor.

4. Esters phénoxypyridylméthyliques de formule I selon la revendication 1, dans lesquels

R représente le reste

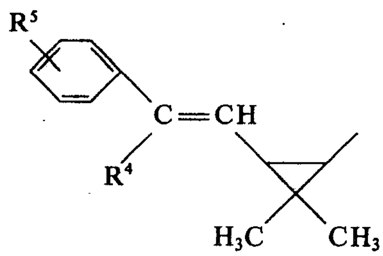

dans lequel

$R^4$ représente le chlore,

$R^5$ représente l'hydrogène ou le chlore (en position para),

R représente en outre le reste

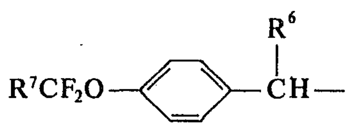

dans lequel

$R^6$ représente un groupe isopropyle, et

$R^7$ représente le fluor,

$R^1$ représente l'hydrogène ou un groupe cyano, et

$R^2$ et $R^3$ représentent l'hydrogène.

5. Produits pesticides caractérisés en ce qu'ils contiennent au moins un ester phénoxypyridylméthylique de formule I.

6. Utilisation des esters phénoxypyridylméthyliques de formule I dans la lutte contre les parasites.

7. Procédé pour combattre les parasites, caractérisé en ce que l'on fait agir les esters phénoxypyridylméthyliques de formule I sur les parasites et/ou leur habitat.

8. Procédé de préparation de produits pesticides, caractérisé en ce que l'on mélange des esters phénoxypyridylméthyliques de formule I avec des diluants et/ou des agents tensio-actifs.